# EUROPEAN PATENT SPECIFICATION

(11) **EP 1 543 768 B1**
(45) Date of publication and mention of the grant of the patent: **26.12.2012**
(21) Application number: 03748547.1
(22) Date of filing: 22.09.2003
(51) Int. Cl.: A61B 5/00, G01N 27/22

(54) **METHOD OF MEASURING WATER CONTENT IN MOUTH AND WATER CONTENT MEASURING INSTRUMENT THEREFORE**
VERFAHREN ZUR MESSUNG DES WASSERGEHALTS IM MUND UND WASSERGEHALT-MESSINSTRUMENT DAFÜR
PROCEDE ET INSTRUMENT PERMETTANT DE MESURER LA TENEUR EN EAU DE LA BOUCHE

(30) Priority: 24.09.2002 JP 2002278189; 16.10.2002 JP 2002302321
(43) Date of publication of application: 22.06.2005
(73) Proprietor: ICST Corporation, Saitama, 338-0001 (JP)
(72) Inventor: KAKINOKI, Yasuaki, Fukuoka-shi, Fukuoka 811-1356 (JP); FURUKAWA, Makoto, Koshigaya-shi, Saitama 343-0846 (JP)
(74) Representative: Loven, Keith James
(86) International application number: PCT/JP2003/012062
(87) International publication number: WO 2004/028359

(56) References cited:
- JP-A- 2 283 313
- JP-A- 8 502 670
- JP-A- 11 083 797
- JP-A- 2001 170 088
- US-A- 4 277 742
- US-A- 4 326 404
- US-A1- 2001 017 053
- US-A1- 2001 027 275
- US-B1- 6 253 098
- US-B1- 6 269 261
- KAKINOKI Y.: 'Koko kansosho 2 daeki bunpitsu teika no mechanism to rinshoteki taio atarashii keisoku kiki yakuzai suibunkei' SHIKAI TENBO, ISHIYAKU PUB., INC. vol. 100, no. 2, 15 August 2002, pages 406 - 407, XP002986483
- KAKINOKI Y.: 'Koko suibunkei moisture checker o kaatsuyo shita kanja eno approach-ho' DENTAL PRODUCT NEWS, YOSHIDA DENTAL TRADE DIST. CO., LTD., GAKUJUTSU EIGYO SUISHIN HONBU GAKUJUTSU HANBAIKA no. 139, 10 March 2003, pages 01 - 03, XP002986484
- KAKINOKI Y.: 'Moisture checker mukasu' ni Tsuite' NIHON SHIKA HYORON KABUSHIKI KAISHA HYORON PUBLISHERS vol. 63, no. 5, 11 May 2003, pages 105 - 109, XP002986485
- KAKINOKI Y.: 'Koko ryoiki ni shojo o arawasu jobiyaku to sono rinshoteki taio koko kansosho' SHIKAI TENBO, ISHIYAKU PUB., INC. vol. 98, no. 4, 15 October 2001, pages 729 - 733, XP002986486

## Description

### TECHNICAL FIELD

The present invention relates to an intra-mouth water content measuring method of measuring a water content in a mouth such as a tongue mucous membrane, a cheek mucous membrane, or a palate, a water content measuring instrument for the method, and a replaceable cover for an intra-mouth insertion unit of the water content measuring instrument. More specifically, the present invention relates to an intra-mouth water content measuring method of measuring a water content in a mouth by bringing a sensor unit of a water content measuring instrument which unit senses the water content into contact via a plastic film or the like with a measurement target region such as a tongue mucous membrane, a cheek mucous membrane, or a palate, the water content measuring instrument used to carry out the method, and a replaceable cover consisting of a plastic cover and covering an intra-mouth insertion unit of the water content measuring instrument.

### BACKGROUND ART

A xerostomia called "dry mouth" is normally thought to be caused by a generalized disease such as diabetes or a renal disease, mouth breathing, smoking, a side effect of a medicine or a treatment, a salivary gland related disease, a central or peripheral nerve disorder, a mental stress or the like. A connection of the dry mouth with dry eye is also considered.

According to reports, elderly people particularly tend to get dry mouth. A Western survey shows that about 40 % of the elderly get dry mouth. In Japan, it is said that about 45% of the elderly, higher than 40%, get dry mouth.

Meanwhile, saliva is secreted from three major salivary glands, i.e., a parotid gland, a submaxillary gland, and a sublingual gland. A saliva production of the salivary glands decreases with age. Salivary secretion of a palatine gland other than the three major salivary glands also decreases with age. Considering these, it may be said that a major cause of the xerostomia is a decrease in salivary secretion with age.

If the elderly gets dry mouth, the following symptoms appear. As a result, the drymouth often influences even appetite, nutritional state, and general medical condition of the elderly.

Mucous membrane disorders and pain in tongue mucous membranes due to a reduction in self-purification, or disappearance of a lubricating function:
- Unstable denture
- Development of fur coating of tongue
- Onset of candidiasis
- Onset or development of carious teeth or periodontitis

Conventionally, a measurement of a water content accompanying a diagnosis of the xerostomia is conducted by a chewing gum test or a Saxon test.

These conventional tests are intended to evaluate an amount of saliva secreted by masticating stimulation. Due to this, considering that many dry mouth patients are associated with a reduction in saliva production when they keep quiet, it is difficult to say that the dry state in the patient's mouth can be accurately measured by the chewing gum test or the Saxon test.

Further, the xerostomia is often discovered by a patient' s complaint of subjective symptoms during a doctor' s consultation. However, many elderly patients take it for granted that the dry state in the mouth cannot be helped due to aging. At worst, the xerostomia is not discovered during this consultation.

It is, therefore, an object of the present invention to provide an intra-mouth water content measuring method which enables easily measuring a salivary production when a person keeps quiet, that is, a water content in a mouth when the person keeps quiet not only in a medical institution such as a hospital but also in the home of the patient oneself or a non-professional person, a water content measuring instrument for the method, and a replacement cover for an intra-mouth insertion unit of the water content measuring instrument.

It is noted that Japanese Patent Application Laid-Open No. 8-94568 introduces technical information on means for measuring a water content of a dielectric or a region that serves as a dielectric against an electrode. In addition, Japanese Patent Application Laid-Open Nos. 2002-172093 and 2003-169788 introduce technical information on means for measuring a water content of a measurement target region on a skin by pressing a sensor against the region. Further, Japanese Utility Model Application Laid-Open No. 63-33453 introduces technical information on means for elastically holding a water content sensor unit by a coil spring, and for keeping a pushing pressure against a measurement target region constant.

US 2001/017053 discloses a device for measuring relative humidity of concrete or gypsum by drilling a hole in the concrete or gypsum, inserting a sleeve, waiting 24 hours, then inserting a probe of the measuring device into the sleeve.

US-A-4277742 discloses a microscopic, integrated circuit type humidity sensor.

US 2001/027275 discloses a sensor to be placed in physical contact with a tooth or tooth tissue for sensing the electrical resistance through enamel, dentine or the tooth tissue, the rate of change of resistance indicating the rate of change of moisture level/porosity, which is a measure of tooth sensitivity.

US 6253098 discloses a replaceable pulse oximeter sensor for measuring blood oxygenation in liquid-filled cavities and/or wounds, combined with a replaceable protective cover to prevent the measurement being compromised by contact with body fluids.

US 6269261 discloses a health care instrument including a replaceable probe and sensor for measuring the oxidation-reduction potential in humans, animals, fish, plants, vegetables or the water, air or earth surrounding such organisms in order to determine the level of active oxygen and thereby the subject's condition in terms of health or growth.

### DISCLOSURE OF THE INVENTION

To attain the technical object, an intra-mouth water content measuring method according to a first aspect of the present invention is an intra-mouth water content measuring method of measuring a water content in a mouth such as the water content of a tongue mucous membrane, a cheek mucous membrane, or a palate, characterized in that the water content is measured by bringing a sensor unit of a water content measuring instrument which unit senses the water content into contact via a plastic film with a measurement target region such as the tongue mucous membrane, the cheek mucous membrane, or the palate, characterized in that the plastic film covers the sensor unit of the water content measuring instrument or the sensor unit and at least a unit to be inserted into the mouth and is replaced at every measurement; wherein the sensor unit that senses the water content includes an electrostatic capacity sensor that measures the water content according to a change in dielectric constant; and wherein the thickness of the plastic film is 2 to 20 µm.

An intra-mouth water content measuring instrument according to a second aspect of the present invention is an intra-mouth water content measuring instrument of measuring a water content in a mouth such as the water content of a tongue mucous membrane, a cheek mucous membrane, or a palate, comprising: a sensor unit that comes into contact via a plastic film with a measurement target region such as the tongue mucous membrane, the cheek mucous membrane, or the palate, and that senses the water content of the measurement target region; and a measuring unit that includes this sensor unit, characterized in that the plastic film covers the sensor unit of the water content measuring instrument or the sensor unit and at least a unit to be inserted into the mouth are covered with a plastic film, and said plastic film is replaceable at every measurement; wherein the sensor unit that senses the water content includes an electrostatic capacity sensor that measures the water content according to a change in dielectric constant; and wherein the thickness of the plastic film is 2 to 20 µm.

The intra-mouth water content measuring instrument according to a third aspect of the present invention is based on the second aspect, and is characterized in that the sensor unit and the measuring unit are constituted integrally with each other, and a probe unit bent into a doglegged shape is provided between the sensor unit and the measuring unit.

The intra-mouth water content measuring instrument according to a fourth aspect of the present invention is based on the second or third aspects, and is characterized in that the plastic film consists of a hydrophobic thermoplastic resin such as polyethylene.

The intra-mouth water content measuring instrument according to a fifth aspect of the present invention is based on any one of the second to the fourth aspects, and is characterized in that the plastic film is a bag.

The intra-mouth water content measuring instrument according to a sixth aspect of the present invention is based on the fifth aspect, and is characterized in that a release paper is bonded onto a one surface of the bag.

The intra-mouth water content measuring instrument according to a seventh aspect of the present invention is based on any one of the second to fourth aspects, and is characterized in that the measuring unit includes a display function of digitally displaying the water content.

According to the invention set forth in the first to seventh aspects of the present invention, a saliva production when a patient keeps quiet, that is, a water content in the mouth when the patient keeps quiet can be easily measured not only in a medical institution such as a hospital but also in the home by the patient oneself or a non-professional person, and the measurement can be used for health care management.

The intra-mouth insertion unit of an intra-mouth water content measuring instrument according to an eighth aspect of the invention is based on any one of the second to seventh aspects, and is characterized in that the plastic film comprises a replaceable cover covering the intra-mouth insertion unit of the intra-mouth water content measuring instrument of measuring a water content in a mouth such as the water content of a tongue mucous membrane, a cheek mucous membrane, or a palate, wherein the replaceable cover is a bag-like cover having one openable end, a peripheral edge of the bag-like cover excluding the open end is welded onto a base sheet, a catch piece is attached to an open end on an upper side surface of the bag-like cover relative to the base sheet, and an adhesion treatment is conducted to a rear surface of the catch piece.

The intra-mouth insertion unit of the intra-mouth water content measuring instrument according to a ninth aspect of the present invention is based on the eighth aspect, and is characterized in that the plastic film that constitutes the bag-like cover consists of a hydrophobic thermoplastic resin such as polyethylene.

The intra-mouth insertion unit of the intra-mouth water content measuring instrument according to a tenth aspect of the present invention is based on the eighth or ninth aspect, and is characterized in that a thickness of the plastic film that constitutes the bag-like cover is set such that a thickness of a base sheet-side part of the bag-like cover is 20 to 50 µm and an upper side part thereof is 2 to 20 µm.

The intra-mouth insertion unit of the intra-mouth water content measuring instrument according to an eleventh aspect of the present invention is based on any one of the eighth to tenth aspects, and is characterized in that the base sheet consists of a paper.

The intra-mouth insertion unit of the intra-mouth water content measuring instrument according to a twelfth aspect of the present invention is based on any one of the eighth to eleventh aspects, and is characterized in that the bag-like cover is formed to be gradually narrower from the open end toward a tip end.

According to the invention set forth in the eighth to twelfth aspects of the present invention, it is possible to attain the replacement cover for the intra-mouth insertion unit of the intra-mouth water content measuring instrument which can dispense with washing and disinfecting the intra-mouth insertion unit at every measurement to measure the water content in the mouth by the intra-mouth water content measuring instrument, which can be easily attached to the intra-mouth insertion unit, which has an excellent contact property for contact with a sensor surface, which is not inadvertently released or taken down by a measurement target person during the measurement, which is simple and hygienic, and which does not cause the measurement target person to feel displeasure.

Furthermore, the replacement cover for the intra-mouth insertion unit of the intra-mouth water content measuring instrument according to a thirteenth aspect of the present invention is based on any one of the eighth to twelfth aspects, and is characterized in that the bag-like cover welded onto the base sheet is hermetically sealed by a surface cover film welded onto the base sheet so as to surround the bag-like cover.

According to the invention set forth in the thirteenth aspect of the present invention, the bag-like cover is hermetically sealed by the base sheet and the surface cover hermetically sealed by the base sheet and the surface cover film, good hygiene can be maintained.

### BRIEF DESCRIPTION OF THE DRAWINGS

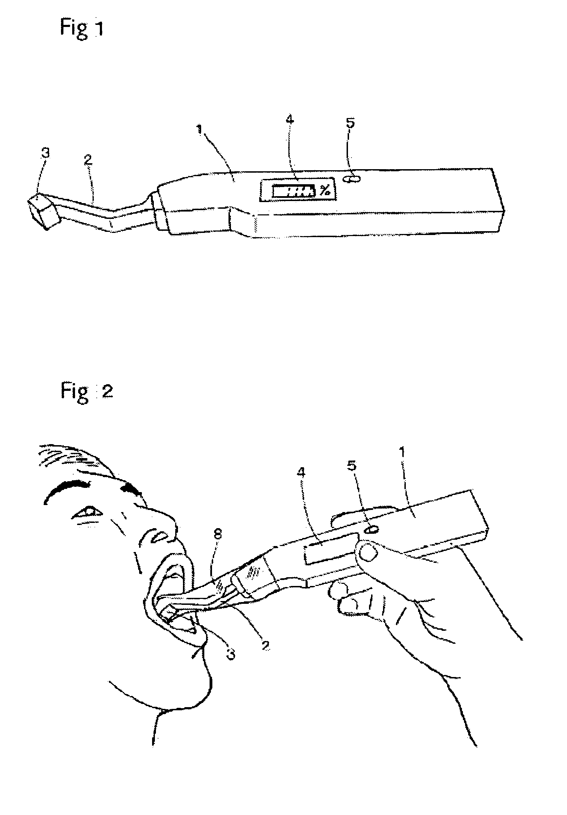

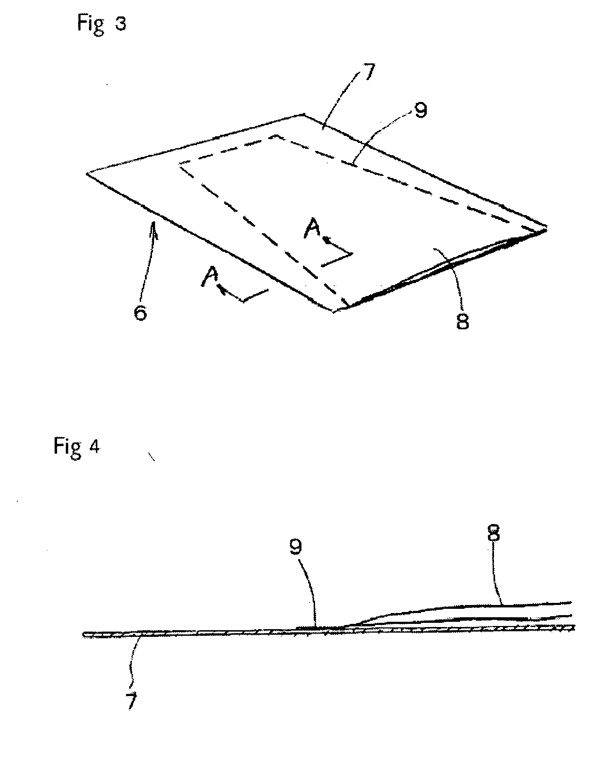

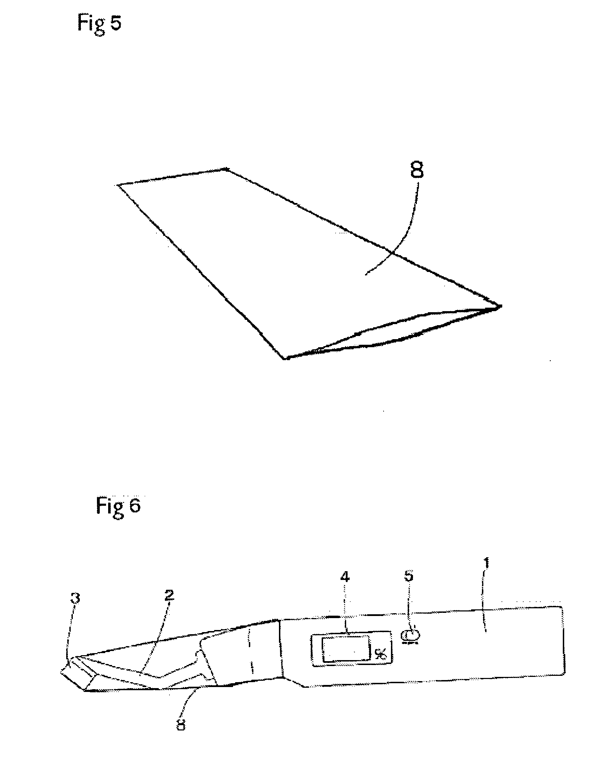

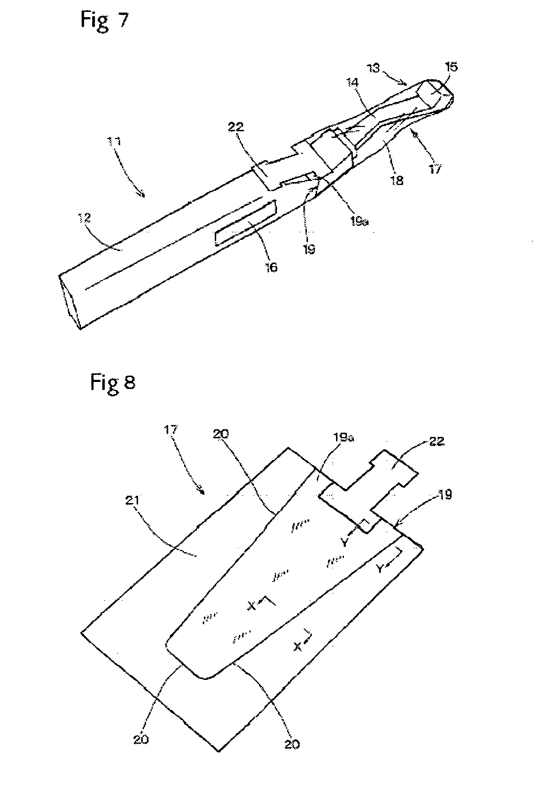

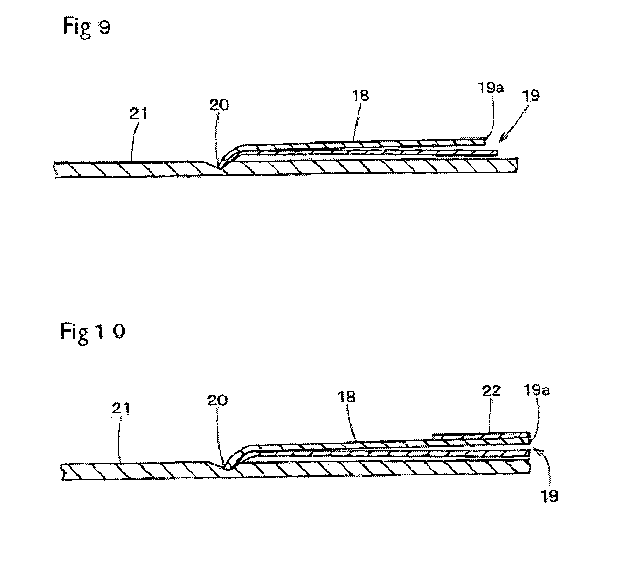

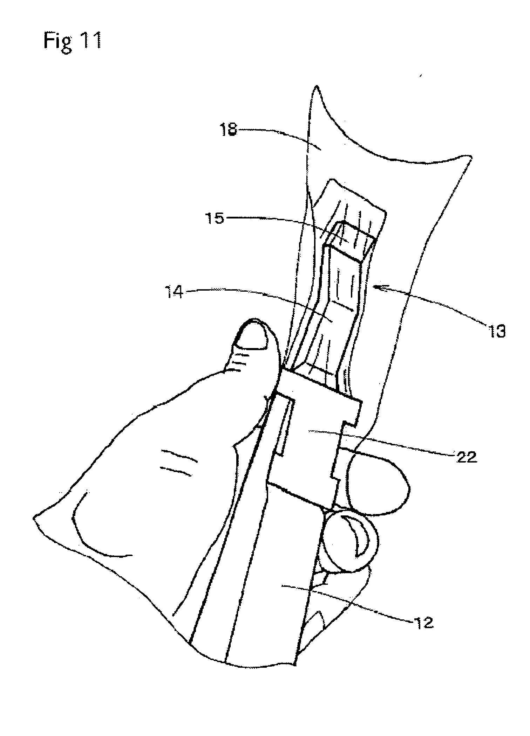

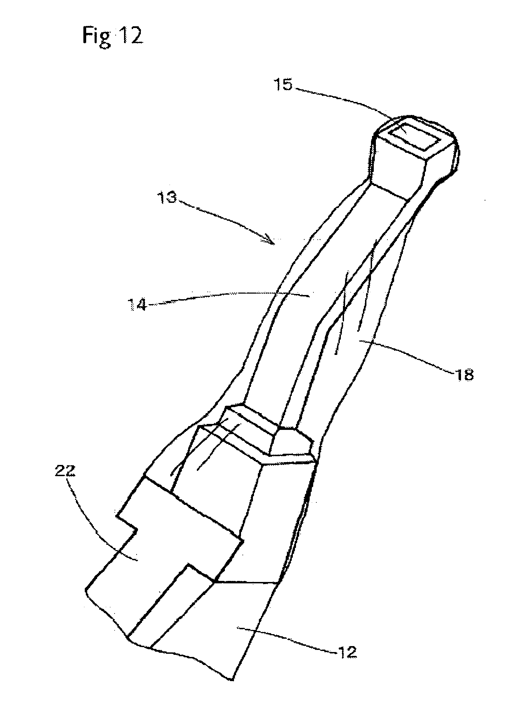

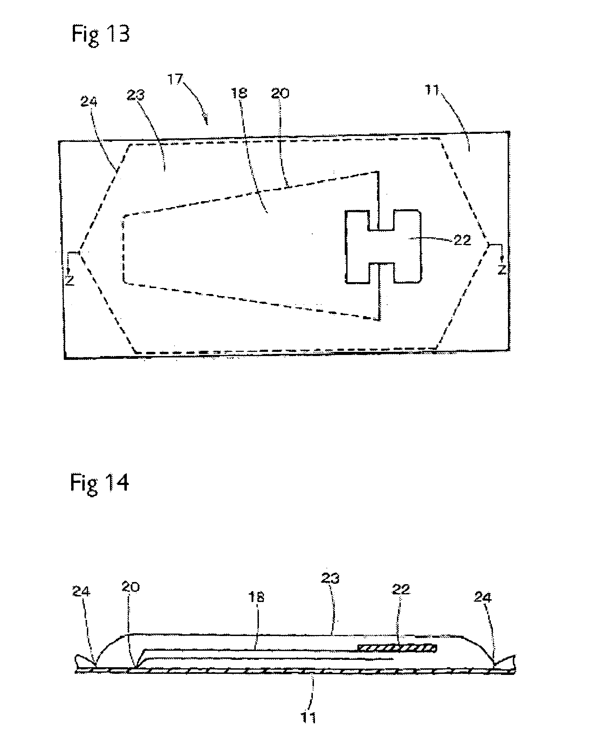

Fig. 1 is a perspective view of an intra-mouth water content measuring instrument according to one embodiment of the present invention; Fig. 2 is a perspective view that depicts a state of measuring a water content in a mouth; Fig. 3 is a perspective view that depicts a state before a plastic film that covers a sensor unit is released; Fig. 4 is a cross-sectional view taken along a line A-A of Fig. 3; Fig. 5 is a perspective view of a released bag-like plastic film; Fig. 6 is a front view of the intra-mouth water content measuring instrument that depicts a state of covering the sensor unit with the bag-like plastic film; Fig. 7 is a perspective view of the intra-mouth water content measuring instrument to which a replacement cover for an intra-mouth insertion unit according to another embodiment of the present invention is attached; Fig. 8 is a perspective view that depicts the replacement cover for the intra-mouth insertion unit of the intra-mouth water content measuring instrument; Fig. 9 is an enlarged cross-sectional view taken along a line X-X of Fig. 8; Fig. 10 is an enlarged cross-sectional view taken along a line Y-Y of Fig. 8; Fig. 11 is a perspective view that depicts a state of attaching the replacement cover for the intra-mouth insertion unit to the intra-mouth insertion unit of the intra-mouth water content measuring instrument; Fig. 12 is a perspective view that depicts a state in which the replacement cover has been attached to the intra-mouth insertion unit; and Figs. 13 and 14 depict a replacement cover for an intra-mouth insertion unit according to yet another embodiment of the present invention, wherein Fig. 13 is a plan view and Fig. 14 is a cross-sectional view taken along a line Z-Z of Fig. 13.

### BEST MODE FOR CARRYING OUT THE INVENTION

The present invention will be described hereinafter in detail with reference to the accompanying drawings.

As shown in Fig. 1, an intra-mouth water content measuring instrument according to the present invention is composed by a measuring unit 1, a probe 2, and a sensor unit 3. The probe 2 is attached to one end of the measuring unit 1, and the sensor unit 3 is provided on a tip end of the probe 2. The measuring unit 1 includes a display unit 4 digitally displaying a measured water content, and provided at a front of the measuring unit 1. Reference symbol 5 denotes a power switch.

A sensor (not shown) that is included in the sensor unit 3 and that senses a water content is an electrostatic capacity sensor that measures the water content according to a change in dielectric constant. A moisture-sensitive high-molecular thin film is formed on a substrate, and an electrostatic capacity of the sensor changes according to the water content. The probe 2 is bent into a doglegged shape in a front view. As shown in Fig. 2, this is intended to facilitate putting the sensor unit 3 against any region of an intra-mouth membrane when the measuring unit 1 is held. A sensitive surface of the sensor unit 3 is sealed by a glass plate or a plastic plate, and a water content-sensitive sensor is provided in an inside surface of the sensor unit 3. The sensor unit 3 measures the water content of a measurement target region such as a tongue mucous membrane, a cheek mucous membrane, or a palate by pressing the sensor unit 3 against the measurement target region. It is, therefore, preferable that the sensor unit 3 includes an elastic holding means for keeping a pushing pressure against the measurement target region constant or a pressure correction circuit. Needless to say, if the sensor unit 3 includes both the elastic holding means and the pressure correction circuit, a measurement accuracy of the sensor unit 3 is further enhanced.

As shown in Figs. 3 and 4, a plastic film 6 that covers the sensor unit 3 is adhesively bonded onto the sensor unit 3 by a welding portion 9 so as to form a bag-like plastic film 8 on one surface of a release paper 7. When the plastic film 6 is used, the bag-like plastic film 8 is released from the release paper 7 at the welding portion 9, as shown in Fig. 5. In addition, as shown in Fig. 6, the bag-like plastic film 8 covers a part of the measuring instrument from the sensor unit 3 to the probe 2, and the water content in the mouth is measured as shown in Fig. 2.

The bag-like plastic film 8 is preferably a thermoplastic resin such as hydrophobic thermoplastic resin, e.g., polyethylene, polypropylene, nylon, or polyvinyl chloride which can be formed thin, and particularly preferably polyethylene. A thickness of the bag-like plastic film 8 is 2 to 20 µm. If the thickness is 2 µm or less, the plastic film 8 may possibly be broken during use. If the thickness exceeds 20 µm, a water content measurement sensitivity of the sensor unit 3 is deteriorated and the measurement accuracy thereof is deteriorated, as well. To improve the sensitivity and the accuracy of the sensor unit 3, therefore, the thickness of the plastic film 8 is more preferably 2 to 15 µm. The bag-like plastic film 8 that covers the sensor unit 3 to the probe 2 is replaced at every measurement. Due to this, even if an intra-mouth water content measurement is repeatedly conducted for a plurality of persons, it is possible to keep the measuring instrument highly hygienic.

As shown in Fig. 7, an intra-mouth water content measuring instrument 11 is composed by amain body unit 12 and an intra-mouth insertion unit 13. The intra-mouth insertion unit 13 is composed by a probe 14 extending from one end of the main body unit 12 in a generally doglegged fashion, and a sensor unit 15 provided on a tip end of the probe 14. The main body unit 12 is an elongated cylinder that is easy to hold by a hand during a measurement. The main body unit 12 includes a display unit 16 that digitally displays a measured water content. The sensor unit 15 is hermetically sealed and a water content sensor is provided inside a sensitive surface thereof. A configuration and a type of the water content sensor are not limited to specific ones. However, in view of the sensor sensitivity, accuracy, stability, and the like, an electrostatic capacity sensor is preferable. A bag-like cover 18 of an intra-mouth insertion unit replacement cover 17 according to the present invention is attached to the intra-mouth insertion unit 13.

As shown in Fig. 8, the intra-mouth insertion unit replacement cover 17 according to the present invention has one end formed into an openable bag. A peripheral edge 20 excluding an open end 19 of the bag-like cover 18 is welded onto a base sheet 21. A catch piece 22 is attached to an open end 19a on an upper surface of the bag-like cover 18 relative to the base sheet 21, and an adhesion treatment is conducted to a rear surface of the catch piece 22. The bag-like cover 18 is formed so as to be gradually narrower in a direction from the open end 19 toward a tip end.

According to the embodiment shown in the drawings, the base sheet 21 is a paper base sheet, and the bag-like cover 18 is formed by superimposing two plastic films on the paper base sheet 21, and thermally sealing the peripheral edge 20 from above. In addition, the bag-like cover 18 is releasably welded and held onto the base sheet 21 by the peripheral edge 20.

As described above, the intra-mouth insertion unit replacement cover 17 according to the present invention is in the form of a thin piece integral with the base sheet 21 when the cover 17 is unused. Therefore, a plurality of covers 17 are put in a packing bag of polyethylene and the like, in piles as a commercial product.

The plastic films that constitute the bag-like cover 18 preferably consist of a thermoplastic resin such as hydrophobic thermoplastic resin, e.g., polyethylene, polypropylene, nylon, or polyvinyl chloride which can be formed thin, and particularly preferably consist of polyethylene. A thickness of the bag-like cover 18 is 2 to 20 µm. If the thickness is 2 µm or less, the cover 18 may possibly be broken during use. If the thickness exceeds 20 µm, the water content measurement sensitivity of the sensor unit 13 is deteriorated and the measurement accuracy thereof is deteriorated, as well. To improve the sensitivity and the accuracy of the sensor unit 13, therefore, the thickness of the bag-like cover 18 is more preferably 2 to 15 µm. The bag-like plastic film 8 covering the sensor unit 13 to the probe 2 is replaced at every measurement. Due to this, even if an intra-mouth water content measurement is repeatedly conducted for a plurality of persons, it is possible to keep the measuring instrument highly hygienic. A thickness of the base sheet 21 is set such that a shape of the bag-like cover 18 can be kept. The catch piece 22 is made of paper and usage directions are preferably written thereon.

To attach the bag-like cover 18 of the intra-mouth insertion unit replacement cover 17 to the intra-mouth insertion unit 13, a user catches and pulls the base sheet 21 and the catch piece 22 in a direction in which the open end 19 of the cover 18 opens, that is, in a direction in which the bag-like cover 18 is released from the base sheet 21. By doing so, the base sheet 21 is released while the open end 19 of the bag-like cover 18 is open.

In this manner, the bag-like cover 18 turns into an open state. Therefore, when the bag-like cover 18 is attached to the intra-mouth insertion unit 13 of the intra-mouth water content measuring instrument 11, an insertion operation for the intra-mouth insertion unit 13 can be performed easily and promptly. Further, the bag-like cover 18 is pulled toward the main body 12 side of the intra-mouth insertion unit 13, a tip end of the bag-like cover 18 is brought into close contact with the sensitive surface of the sensor unit 15, and the catch piece 22 is adhesively attached to the main body 12 so as to keep the adhesive state. After attaching the bag-like cover 18 to the intra-mouth insertion unit 13, the intra-mouth insertion unit 13 is inserted into the mouth, the sensor unit 15 is pressed against a measurement target region such as a tongue mucous membrane, a cheek mucous membrane, or a palate to measure the water content of the measurement target region. The measured water content is digitally displayed on the display unit 16. The bag-like cover 18 is replaced by a new one at every water content measurement.

The intra-mouth insertion unit replacement cover shown in Figs. 13 and 14 is constituted so that the bag-like cover 18 welded onto the base sheet 21 is covered and hermetically sealed with a surface cover film 23 welded onto the base sheet 21 so as to surround the cover 18. A welding portion 24 of the surface cover film 23 welded to the base sheet 21 is an easy-open seal foamed with a heat seal so as to facilitate releasing the surface cover film 23 from the base sheet 21. The surface cover film 23 is a polypropylene film which is sterilizable using ethylene oxide gas (EOG), and has a thickness of 15 to 50 µm. The bag-like cover 19 according to this embodiment is harder to break when being released from the base sheet 21 if a base sheet-side part of the cover 18 is thicker. Therefore, it is appropriate to set a thickness of the base sheet 21-side part of the cover 18 at 20 to 50 µm (preferably 20 to 30 µm) and a thickness of an upper side part thereof at 2 to 20 µm (preferably 8 to 15 µm). An optimum thickness of the base sheet 21-side part of the bag-like cover 18 is 20 to 30 and an optimum thickness of the upper-side part thereof is 8 to 15 µm.

### INDUSTRIAL APPLICABILITY

The intra-mouth water content measuring method and the water content measuring instrument for the method according to the present invention enable easily measuring a saliva production when a measurement target person keeps quiet, that is, a water content in the mouth when the person keeps quiet. Therefore, it is highly likely that the intra-mouth water content measuring method and the water content measuring instrument for the method according to the present invention are widely used not only for internal medicine for generalized diseases such as diabetes and renal diseases, and medical care for the elderly, but also dental and oral care as well as everyday healthcare in health promotion facilities, elderly day care centers, homes, and the like. In addition, it is expected to expand an available area of the instrument and the method particularly to a checkup of Sjögren' s syndrome (a kind of a collagen disease, e.g., rheumatoid arthritis or diabetes) in internal medicine.

## Claims

1. An intra-mouth water content measuring method of measuring a water content in a mouth such as the water content of a tongue mucous membrane, a cheek mucous membrane, or a palate, wherein the water content is measured by bringing a sensor unit (3) of a water content measuring instrument (1) which unit senses the water content into contact via a plastic film (8) with a measurement target region such as the tongue mucous membrane, the cheek mucous membrane, or the palate, **characterized in that** the plastic film (8) covers the sensor unit (3) of the water content measuring instrument (1) or the sensor unit (3) and at least a unit (2) to be inserted into the mouth and is replaced at every measurement; wherein the sensor unit (3) that senses the water content includes an electrostatic capacity sensor that. measures the water content according to a change in dielectric constant; and wherein the thickness of the plastic film is 2 to 20 µm.

2. An intra-mouth water content measuring instrument (1) for measuring a water content in a mouth such as the water content of a tongue mucous membrane, a cheek mucous membrane, or a palate, comprising a sensor unit (3) that comes into contact via a plastic film (8) with a measurement target region such as the tongue mucous membrane, the cheek mucous membrane, or the palate, and that senses the water content of the measurement target region, a measuring unit (1) that includes this sensor unit (3) a plastic film (8) and a unit (2) to be inserted into the mouth, **characterized in that** the plastic film (8) covers the sensor unit (3) of the water content measuring instrument, or the sensor unit and at least said unit (2) to be inserted into the mouth are covered with a plastic film, and said plastic film is replaceable at every measurement; wherein the sensor unit that senses the water content includes an electrostatic capacity sensor that measures the water content according to a change in dielectric constant; and wherein the thickness of the plastic film is 2 to 20 µm.

3. The intra-mouth water content measuring instrument according to claim 2, wherein the sensor unit and the measuring unit are constituted integrally with each other, and a probe unit bent into a doglegged shape is provided between the sensor unit and the measuring unit.

4. The intra-mouth water content measuring instrument according to claim 2 or 3, wherein the plastic film consists of a hydrophobic thermoplastic resin such as polyethylene.

5. The intra-mouth water content measuring instrument according to any one of claims 2 to 4, wherein the plastic film is a bag.

6. The intra-mouth water content measuring instrument according to claim 5, wherein a release paper is bonded onto a one surface of the bag.

7. The intra-mouth water content measuring instrument according to any one of claims 2 to 4, wherein the measuring unit includes a display function of digitally displaying the water content.

8. An intra-mouth water content measuring instrument as claimed in claim 5, wherein the bag has one openable end, a peripheral edge of the bag excluding the open end is welded onto a base sheet, a catch piece is attached to an open end on an upper side surface of the bag relative to the base sheet, and an adhesion treatment is conducted to a rear surface of the catch piece.

9. The intra-mouth water content measuring instrument according to claim 8, wherein a thickness of the plastic film that constitutes the bag is set such that a thickness of a base sheet-side part of the bag is 20 to 50 µm and an upper side part thereof is 2 to 20 µm.

10. The intra-mouth water content measuring instrument according to claim 8 or 9, wherein the base sheet consists of paper.

11. The intra-mouth water content measuring instrument according to any one of claims 8 to 10, wherein the bag is formed to be gradually narrower from the open end toward a tip end.

12. The intra-mouth water content measuring instrument according to any one of claims 8 to 11, wherein the bag welded onto the base sheet is hermetically sealed by a surface cover film welded onto the base sheet so as to surround the bag.

## Patentansprüche

1. Intraorales Wassergehaltsmessverfahren zum Messen des Wassergehalts im Mund wie beispielsweise des Wassergehalts der Zungenschleimhaut, der Wangenschleimhaut oder des Gaumens, wobei der Wassergehalt gemessen wird, indem eine Sensoreinheit (3) eines Wassergehaltsmessinstruments (1), die den Wassergehalts erfasst, über eine Kunststofffolie (8) in Kontakt mit einer zu messenden Zielregionen wie der Zungenschleimhaut, der Wangenschleimhaut oder des Gaumens gebracht wird, **dadurch gekennzeichnet, dass** die Kunststofffolie (8) die Sensoreinheit (3) des Wassergehaltsmessinstruments (1) oder die Sensoreinheit (3) und zumindest eine Einheit (2), die in den Mund einzuführen ist, bedeckt und bei jeder Messung ersetzt wird; wobei die Sensoreinheit (3), die den Wassergehalt misst, einen elektrostatischen Kapazitätssensor aufweist, der den Wassergehalt in Abhängigkeit einer Veränderung der Dielektrizitätskonstanten misst; und wobei die Dicke der Kunststofffolie 2 bis 20 µm beträgt.

2. Intraorales Wassergehaltsmessinstrument (1) zum Messen des Wassergehalts im Mund wie beispielsweise des Wassergehalts der Zungenschleimhaut, der Wangenschleimhaut oder des Gaumens, umfassend eine Sensoreinheit (3), die über eine Kunststofffolie (8) in Kontakt mit einer zu messenden Zielregionen wie der Zungenschleimhaut, der Wangenschleimhaut oder des Gaumens gelangt, und die den Wassergehalt der Zielregion misst, eine Messeinheit (1), die diese Sensoreinheit (3) aufweist, eine Kunststofffolie (8) und eine in den Mund einzuführende Einheit (2), **dadurch gekennzeichnet, dass** die Kunststofffolie (8) die Sensoreinheit (3) des Wassergehaltsmessinstruments bedeckt oder die Sensoreinheit und zumindest besagte Einheit (2), die in den Mund einzuführen ist, mit einer Kunststofffolie bedeckt sind, und besagte Kunststofffolie für jede Messung ersetzbar ist; wobei die Sensoreinheit, die den Wassergehalt erfasst, einen elektrostatischen Kapazitätssensor aufweist, der den Wassergehalt in Abhängigkeit einer Veränderung der Dielektrizitätskonstanten misst; und wobei die Dicke der Kunststofffolie 2 bis 20 µm beträgt.

3. Intraorales Wassergehaltsmessinstrument nach Anspruch 2, bei dem die Sensoreinheit und die Messeinheit integral miteinander ausgebildet sind und eine gegenläufig gebogene Fühlereinheit zwischen der Sensoreinheit und der Messeinheit vorgesehen ist.

4. Intraorales Wassergehaltsmessinstrument nach Anspruch 2 oder 3, bei dem die Kunststofffolie aus einem hydrophoben thermoplastischen Kunststoff wie Polyethylen besteht.

5. Intraorales Wassergehaltsmessinstrument nach einem der Ansprüche 2 bis 4, bei dem die Kunststofffolie eine Tasche ist.

6. Intraorales Wassergehaltsmessinstrument nach Anspruch 5, bei dem ein Trennpapier an einer Oberfläche der Tasche aufgeklebt ist.

7. Intraorales Wassergehaltsmessinstrument nach einem der Ansprüche 2 bis 4, bei dem die Messeinheit eine Anzeigefunktion zur digitalen Anzeige des Wassergehalts aufweist.

8. Intraorales Wassergehaltsmessinstrument nach Anspruch 5, bei dem die Tasche ein öffenbares Ende aufweist, ein umlaufender Rand der Tasche mit Ausnahme des offenen Endes auf eine Unterlage aufgeschweißt ist, ein Fangstück an einem offenen Ende an einer Oberseite der Tasche, bezogen auf die Unterlage, angebracht ist, und eine Haftbehandlung an der Rückseite des Fangstücks ausgeführt ist.

9. Intraorales Wassergehaltsmessinstrument nach Anspruch 8, bei dem die Dicke der Kunststofffolie, welche die Tasche bildet, so gewählt ist, dass die Dicke auf der Seite der Unterlage 20 bis 50 µm beträgt und auf der Oberseite 2 bis 20 µm beträgt.

10. Intraorales Wassergehaltsmessinstrument nach Anspruch 8 oder 9, bei dem die Unterlage aus Papier ist.

11. Intraorales Wassergehaltsmessinstrument nach einem der Ansprüche 8 bis 10, bei dem die Tasche sich vom offenen Ende zu einer Spitze fortschreitend verjüngt.

12. Intraorales Wassergehaltsmessinstrument nach einem der Ansprüche 8 bis 11, bei dem die Tasche, die auf die Unterlage aufgeschweißt ist, durch eine Hülle, die auf die Unterlage aufgeschweißt ist und die Tasche umgibt, hermetisch abgedichtet ist.

## Revendications

1. Procédé de mesure de teneur en eau d'une bouche consistant à mesurer une teneur en eau dans une bouche, telle que la teneur en eau d'une membrane muqueuse de la langue, d'une membrane muqueuse de la joue, ou d'un palais, la teneur en eau étant mesurée par la mise d'une unité de capteur (3) d'un instrument de mesure de teneur en eau (1), laquelle unité détecte la teneur en eau, en contact, par l'intermédiaire d'un film de matière plastique (8), avec une région cible de mesure, telle que la membrane muqueuse de la langue, la membrane muqueuse de la joue, ou le palais, **caractérisé par le fait que** le film de matière plastique (8) recouvre l'unité de capteur (3) de l'instrument de mesure de teneur en eau (1) ou l'unité de capteur (3) et au moins une unité (2) devant être introduite dans la bouche et est remplacé à chaque mesure ; l'unité de capteur (3) qui détecte la teneur en eau comprenant un capteur de capacité électrostatique qui mesure la teneur en eau en fonction d'un changement de la constante diélectrique ; et l'épaisseur du film de matière plastique étant de 2 à 20 µm.

2. Instrument (1) de mesure de teneur en eau d'une bouche destiné à mesurer une teneur en eau dans une bouche, telle que la teneur en eau d'une membrane muqueuse de la langue, d'une membrane muqueuse de la joue, ou d'un palais, comprenant une unité de capteur (3) qui vient en contact, par l'intermédiaire d'un film de matière plastique (8), avec une région cible de mesure, telle que la membrane muqueuse de la langue, la membrane muqueuse de la joue, ou le palais, et qui détecte la teneur en eau de la région cible de mesure, une unité de mesure (1) qui comprend cette unité de capteur (3), un film de matière plastique (8) et une unité (2) devant être introduite dans la bouche, **caractérisé par le fait que** le film de matière plastique (8) recouvre l'unité de capteur (3) de l'instrument de mesure de teneur en eau, ou l'unité de capteur et au moins ladite unité (2) devant être introduite dans la bouche sont recouvertes d'un film de matière plastique, et ledit film de matière plastique étant remplaçable à chaque mesure ; l'unité de capteur qui détecte la teneur en eau comprenant un capteur de capacité électrostatique qui mesure la teneur en eau en fonction d'un changement de la constante diélectrique ; et l'épaisseur du film de matière plastique étant de 2 à 20 µm.

3. Instrument de mesure de teneur en eau d'une bouche selon la revendication 2, dans lequel l'unité de capteur et l'unité de mesure sont constituées d'un seul tenant l'une à l'autre, et une unité de sonde de forme coudée est disposée entre l'unité de capteur et l'unité de mesure.

4. Instrument de mesure de teneur en eau d'une bouche selon l'une des revendications 2 ou 3, dans lequel le film de matière plastique est constitué d'une résine thermoplastique hydrophobe, telle que le polyéthylène.

5. Instrument de mesure de teneur en eau d'une bouche selon l'une quelconque des revendications 2 à 4, dans lequel le film de matière plastique est un sac.

6. Instrument de mesure de teneur en eau d'une bouche selon la revendication 5, dans lequel un papier antiadhésif est collé sur une surface du sac.

7. Instrument de mesure de teneur en eau d'une bouche selon l'une quelconque des revendications 2 à 4, dans lequel l'unité de mesure comprend une fonction d'affichage pour l'affichage numérique de la teneur en eau.

8. Instrument de mesure de teneur en eau d'une bouche selon la revendication 5, dans lequel le sac a une extrémité apte à être ouverte, un bord périphérique du sac à l'exception de l'extrémité ouverte est soudé sur une feuille de base, un élément de saisie est fixé à une extrémité ouverte sur une surface latérale supérieure du sac par rapport à la feuille de base, et un traitement d'adhésion est conduit sur une surface arrière de l'élément de saisie.

9. Instrument de mesure de teneur en eau d'une bouche selon la revendication 8, dans lequel une épaisseur du film de matière plastique qui constitue le sac est définie de telle sorte qu'une épaisseur d'une partie côté feuille de base du sac est de 20 à 50 µm et d'une partie latérale supérieure de celui-ci est de 2 à 20 µm.

10. Instrument de mesure de teneur en eau d'une bouche selon l'une des revendications 8 ou 9, dans lequel la feuille de base est constituée de papier.

11. Instrument de mesure de teneur en eau d'une bouche selon l'une quelconque des revendications 8 à 10, dans lequel le sac est formé pour être progressivement plus étroit à partir de l'extrémité ouverte vers une extrémité de pointe.

12. Instrument de mesure de teneur en eau d'une bouche selon l'une quelconque des revendications 8 à 11, dans lequel le sac soudé sur la feuille de base est scellé hermétiquement par un film de recouvrement de surface soudé sur la feuille de base de façon à entourer le sac.
